Europäisches Patentamt

European Patent Office

Office européen des brevets

(11) Numéro de publication: **0 140 723**
A1

## (12) DEMANDE DE BREVET EUROPEEN

(21) Numéro de dépôt: 84401660.0

(22) Date de dépôt: 10.08.84

(51) Int. Cl.⁴: **C 12 M 1/10,** C 12 M 1/16,
C 12 P 7/48, C 12 N 1/16
//
(C12P7/48, C12R1:685),
(C12P7/48, C12R1:73),
(C12P7/48, C12R1:645)

(30) Priorité: 11.08.83 FR 8313201

(71) Demandeur: MULTIBIO, Société Anonyme dite, 45, rue de la Chaussée d'Antin, F-75009 Paris (FR)

(43) Date de publication de la demande: 08.05.85
Bulletin 85/19

(72) Inventeur: Legesgue, Yves, Le Relais 34, Grande Rue Commeny, F-95450 Vigny (FR)

(84) Etats contractants désignés: AT BE CH DE GB IT LI NL SE

(74) Mandataire: Jolly, Jean-Pierre et al, Cabinet BROT et JOLLY 83, rue d'Amsterdam, F-75008 Paris (FR)

(54) Procédé et installation pour la fermentation en milieu solide de substrats glucidiques.

(57) L'invention concerne un procédé de fermentation en milieu solide de substrats glucidiques.

Selon l'invention, on mélange des déchets glucidiques solides d'origine agricole à l'état particulaire et des substrats liquids riches en glucides, également d'origine agricole, avec des additifs nutritifs et des souches de microorganismes aptes à provoquer la fermentation desdits déchets, on amène le pH du mélange à une valeur inférieure à 7, on introduit le mélange résultant dans une enceinte de fermentation cylindrique entraînée en rotation autour de son axe et équipée de moyens d'agitation dudit mélange, on humidifie ledit mélange à l'intérieur de ladite enceinte et on y maintient la température à une valeur appropriée au microorganisme utilisé, on sépare les matières fermentées solides ou pâteuses du jus de fermentation et on récupère, d'une part, ledit jus de fermentation contenant les métabolites que l'on purifie et l'on concentre, et, d'autre part, lesdites matières solides ou pâteuses.

## Procédé et installation pour la fermentation en milieu solide de substrats glucidiques

La présente invention a trait au domaine de la valorisation de sous produits d'origine agro-alimentaire. Elle concerne plus particulièrement la fermentation en milieu solide de substrats glucidiques.

La croissance démographique mondiale se traduit par une augmentation de plus en plus forte de la consommation d'aliments carnés et, par voie de conséquence, par une intensification de l'élevage et un développement des sources de protéines alimentaires.

On s'accorde à penser que l'accroissement des productions de protéines d'origine végétale ne suffira pas à couvrir ces besoins et qu'il est nécessaire d'envisager les possibilités de production de protéines par des microorganismes tels que les bactéries, les levures et les champignons.

En outre, de nombreux produits organiques peuvent être produits par culture de microorganismes sur des substrats appropriés. Les divers produits industriels élaborés par les microorganismes peuvent être regroupés en plusieurs classes:

- solvants industriels,
- acides organiques,
- amino-acides et nucléotides,
- antibiotiques,
- polysaccharides,
- autres composés.

Parmi les nombreux substrats utilisables par les microorganismes, les substrats glucidiques d'origine agro-alimentaire présentent l'avantage d'être à la fois abondants et théoriquement inépuisables puisqu'ils sont générés en permanence grâce à la photosynthèse.

L'utilisation des résidus agricoles disponibles en quantités importantes, voire gênantes, peut permettre leur valorisation tout en supprimant des sources de pollution.

L'invention concerne plus spécialement le traitement par fermentation de mélasses, de jus de canne concentrés, de jus de betterave, de bagasse, de paille, de pulpes de betterave ou de substrats glucidiques similaires.

Le but essentiel de l'invention est de proposer un procédé de fermentation aérobie plus économique pour la production d'aliments riches en protéines, à partir de substrats glucidiques.

Un autre but de l'invention est de permettre la séparation dans certaines conditions de produits de métabolisme valorisables en obtenant, en même temps que le métabolite recherché, une masse riche en protéines, utilisable comme aliment du bétail.

Un autre but de l'invention est de fournir un type de fermenteur utilisant les microorganismes fixés sur supports solides et assurant dans des conditions optimales le déroulement des réactions de fermentation, en milieu solide ou semi-solide.

A cet effet, l'invention a pour objet un procédé de fermentation en milieu solide de substrats glucidiques, caractérisé en ce que l'on mélange des déchets glucidiques solides d'origine agricole à l'état particulaire et des substrats liquides riches en glucides, également d'origine agricole, avec des additifs nutritifs et des souches de microorganismes aptes à provoquer la fermentation desdits déchets,en ce que l'on amène le pH du mélange à une valeur inférieure à 7, en ce que l'on introduit le mélange résultant dans une enceinte de fermentation cylindrique entraînée en rotation autour de son axe et équipée de moyens d'agitation dudit mélange, en ce que l'on humidifie ledit mélange à l'intérieur de ladite enceinte et en ce que l'on y maintient la température à une valeur appropriée au microorganisme utilisé, en ce qu'après fermentation, on sépare les matières fermentées solides ou pâteuses du jus de fermentation, en ce que l'on récupère, d'une part, ledit jus de fermentation contenant les métabolites que l'on purifie et l'on concentre, et, d'autre part, lesdites matières solides ou pâteuses.

Les matières solides ou pâteuses récupérées peuvent être séchées et broyées en vue de l'obtention d'une poudre à haute teneur en protéines, ou être additionnées en l'état à des produits alimentaires tels que des céréales.

Les déchets solides d'origine agricole sont, par exemple, de la bagasse, de la paille ou de la pulpe de betterave, tandis que les substrats liquides sont des mélasses, du jus de canne ou du jus de betterave.

Les souches de microorganismes utilisées sont de préférence fixées sur des supports argileux en poudre.

L'invention a également pour objet une installation pour la fermentation en milieu solide d'un mélange de substrats glucidiques solides d'origine agricole, de substrats liquides d'origine agricole et d'additifs, caractérisée en ce qu'elle comprend: une enceinte de fermentation cylindrique entraînée en rotation autour de son axe et équipée intérieurement d'une vis sans fin périmétrale, de préférence à spires inclinées; des moyens pour introduire ledit mélange à l'extrémité inférieure de ladite enceinte; des moyens pour évacuer le mélange fermenté à l'extrémité supérieure de ladite enceinte, des moyens pour pulvériser de l'eau à l'intérieur de ladite enceinte et des moyens pour y maintenir la température à un niveau prédéterminé.

Les moyens pour maintenir la température de ladite enceinte à un niveau prédéterminé peuvent comprendre avantageusement un système d'arrosage de la partie extérieure de ladite enceinte.

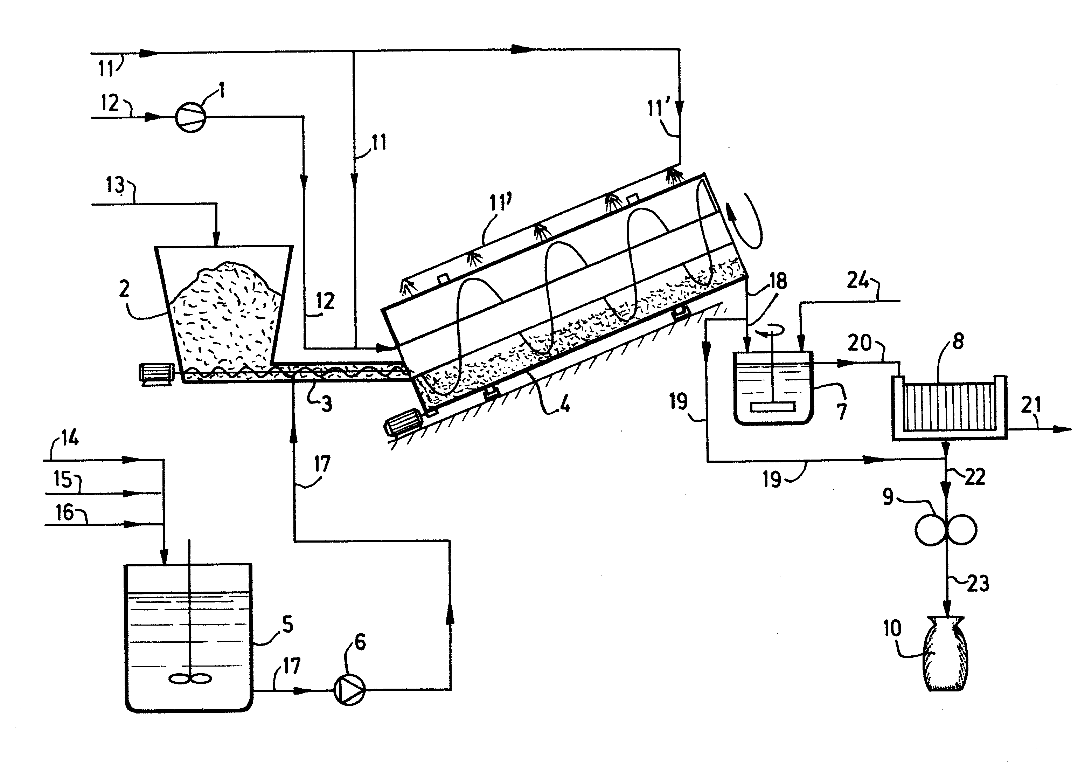

Le dessin annexé illustre sous forme schématique une installation conforme à l'invention.

Les matières premières solides broyées telles que bagasse, paille, pulpe de betterave sont acheminées en 13 dans la trémie de stockage 2 puis, par l'intermédiaire du transporteur-doseur 3, introduites dans le réacteur de fermentation 4.

Les matières premières liquides comme les mélasses, jus de betterave,etc. sont amenées par la ligne 14 dans le réservoir de préparation-stockage 5,dans lequel sont également introduits les microorganismes, de préférence fixés sur un support argileux en poudre, par 15, ainsi que les éléments nutritifs, notamment azote et phosphore, par 16, dans les proportions adéquates.

Le milieu liquide ainsi préparé est dosé à l'aide de la pompe doseuse 6 et introduit dans le transporteur 3 où s'effectue le mélange de ces deux milieux, solide et liquide, en proportions imposées par les paramètres nécessaires dans le réacteur 4.

Le réacteur de fermentation 4 est constitué par un cylindre entraîné en rotation, équipé à l'intérieur d'une vis sans fin, périmétrale, de préférence à spires inclinées, en vue d'augmenter la surface de contact air/mélange à fermenter. Le réacteur 4 est incliné sur l'horizontale, de préférence d'un angle de 45°, l'admission du mélange à traiter se faisant à son extrémité la plus basse. En vue de maintenir un degré hygrométrique satisfaisant dans le réacteur 4, de l'eau, amenée par la ligne 11, y est atomisée par de l'air, acheminé par le ventilateur 1 dans la ligne 12. De l'eau arrose également l'extérieur du réacteur 4, par la ligne 11', en vue de maintenir dans le réacteur la température désirée.

La rotation du réacteur permet le brassage et l'aération du milieu en fermentation. La vitesse de rotation, combinée avec l'angle d'inclinaison du réacteur et celui de la vis intérieure, favorise le retournement des matériaux traités et un contact convenable avec l'oxygène, ainsi que la progression des matières en fermentation vers l'extrémité haute du réacteur dans le temps prévu pour l'obtention du rendement optimum de fermentation.

Si les matériaux sortant du réacteur 4 contiennent de l'acide citrique, ou ont une haute teneur en métabolites, on les achemine par la ligne 18 dans un récipient 7, où l'acide ou les produits intéressants sont extraits par percolation, puis par la ligne 20 vers un filtre-presse ou filtre à vide 8. La phase liquide est évacuée par la ligne 8 vers un ensemble de purification et de concentration, tandis que les matières solides, évacuées par la ligne 22, sont séchées en 9, puis broyées et ensachées en 10.

Eventuellement, les matières sortant du réacteur 4 peuvent être acheminées telles quelles par la ligne 19, ou après un séchage partiel en 9, vers le poste d'ensachage 10, où elles sont mélangées à des céréales ou d'autres aliments.

A titre d'exemple de mise en oeuvre de l'invention, on citera la fermentation en milieu solide, afin d'obtenir des aliments riches en protéines, d'un mélange de bagasse de canne broyée ayant la composition moyenne suivante:

- humidité: 49-51 % (par rapport au poids sec),
- cellulose: 30 à 40 %,
- pentosanes: 22 à 30 %,
- lignine: 18 à 22 %,
- cendres: 1 à 4 %;

et de mélasse de canne de composition:

- eau: 25 %,
- saccharose: 36 %,
- glucose: 7 %,
- fructose: 9 %,
- autres matières organiques: 14,5 %,
- cendres: 8,5 %.

L'humidité du mélange ainsi réalisé est de 55 à 60 %.

On ajoute les éléments nutritifs, notamment azote (sous forme d'un mélange d'urée et de sels d'ammonium) et phosphore (sous forme de phosphate) pour obtenir un rapport: carbone assimilable / N/ P proche de 100/ 5/ 1.

On utilise comme microorganisme Trichoderma album ou une autre souche possédant de bonnes propriétés cellulotyques. Trichoderma album est non seulement riche en protéines, mais aussi très pauvre en acides nucléiques et pauvre en parois cellulaires. Elle ne produit ni toxines ni hormones.

Pour obtenir une bonne croissance, on utilise une concentration d'inoculum d'environ $2.10^7$ spores/g de substrat, soit sous forme libre, soit fixé sur des supports argileux en poudre.

Le pH du milieu est un facteur essentiel dans ce type de fermentation et il doit être suffisamment acide dès le début pour éliminer les risques de contamination. Un moyen simple pour tamponner le milieu est d'apporter l'azote sous forme d'un mélange de sulfate d'ammonium et d'urée en obtenant un pH initial du produit voisin de 4,5. Le milieu en fermentation doit être maintenu à une température voisine de 35°C.

La masse fermentée peut être soit séchée à moins de 40 % d'eau, puis broyée et ensachée, soit mélangée avec des céréales, évitant ainsi le séchage. Le produit aurait alors une humidité de 40 % et serait utilisable en l'état.

Un autre exemple d'application de l'invention est la production simultanée d'acide citrique et d'aliment riche en protéines.

En partant des mêmes substrats que ceux cités précédemment, on utilise des souches comme Saccharomycopsis lipolytica, Candida lipolytica ou Aspergillus niger.

Le pH doit être maintenu autour de 4,7 en début de fermentation.Le pH chute ensuite à environ 2, du fait de l'accumulation d'acide citrique. La température dans la masse à fermenter ne doit pas dépasser 28°C.

L'excrétion d'acide citrique devient importante pour des concentrations très faibles en ions ammonium.

La fin de la phase exponentielle de croissance coïncide avec la disparition quasi-complète de l'azote assimilable du milieu de culture.

On observe, en fin de croissance, l'apparition des acides citrique et isocitrique dans le milieu. Leur concentration augmente alors rapidement; la culture est dite en phase d'excrétion. La durée de la phase linéaire d'excrétion est d'environ 70 à 80 h.

En ajoutant au milieu du méthanol (jusqu'à 4 % en poids du substrat assimilable), on permet aux microorganismes d'utiliser des substrats contenant des concentrations élevées de zinc, de fer ou de manganèse.

Comme indiqué ci-dessus, l'extraction de l'acide citrique à partir de la masse fermentée s'effectue par percolation dans le récipient 7, suivie d'une filtration dans le filtre presse 8.

Les gâteaux obtenus de matière solide provenant du filtrage sont séchés, broyés et ensachés. Ils constituent des aliments riches en protéines pour le bétail.

Revendications

1. Procédé de fermentation en milieu solide de substrats glucidiques, caractérisé en ce que l'on mélange des déchets glucidiques solides d'origine agricole à l'état particulaire et des substrats liquides riches en glucides, également d'origine agricole, avec des additifs nutritifs et des souches de microorganismes aptes à provoquer la fermentation desdits déchets, en ce que l'on amène le pH du mélange à une valeur inférieure à 7, en ce que l'on introduit le mélange résultant dans une enceinte de fermentation cylindrique entraînée en rotation autour de son axe et équipée de moyens d'agitation dudit mélange, en ce que l'on humidifie ledit mélange à l'intérieur de ladite enceinte et en ce que l'on y maintient la température à une valeur appropriée au microorganisme utilisé, en ce qu'après fermentation, on sépare les matières fermentées solides ou pâteuses du jus de fermentation, et en ce que l'on récupère, d'une part, ledit jus de fermentation contenant les métabolites, que l'on purifie et l'on concentre, et, d'autre part, lesdites matières solides ou pâteuses.

2. Procédé selon la revendication 1, caractérisé en ce que les matières solides ou pâteuses récupérées sont séchées et broyées en vue de l'obtention d'une poudre à haute teneur en protéines.

3. Procédé selon la revendication 1, caractérisé en ce que les matières solides ou pâteuses récupérées sont additionnées en l'état à des produits alimentaires tels que des céréales.

4. Procédé selon l'une des revendications 1 à 3, caractérisé en ce que les déchets solides d'origine agricole sont choisis dans le groupe comprenant la bagasse, la paille et la pulpe de betterave, tandis que les substrats liquides sont choisis dans le groupe comprenant les mélasses, le jus de canne et le jus de betterave.

5. Procédé selon l'une des revendications 1 à 4, caractérisé en ce que les souches de microorganismes sont fixées sur des supports argileux en poudre.

6. Procédé selon l'une des revendications 1 à 5, caractérisé en ce que l'on utilise comme souche de microorganismes Trichoderma album, en ce que le pH initial du mélange introduit dans l'enceinte de fermentation est égal à environ 4,5, et en ce que l'on maintient la température à l'intérieur de l'enceinte de fermentation à environ 35°C.

7. Procédé selon la revendication 6, caractérisé en ce que, pour régler le pH initial du mélange, on utilise un mélange de sulfate d'ammonium et d'urée.

8. Procédé selon l'une des revendications 1 à 5, caractérisé en ce que l'on utilise comme souche de microorganismes Saccharomycopsis lipolytica, Candida lipolytica ou Aspergillus niger, en ce que le pH initial du mélange introduit dans l'enceinte de fermentation est égal à environ 4,7, en ce que l'on maintient la température à l'intérieur de l'enceinte de fermentation à une valeur inférieure ou égale à environ 28°C et en ce que l'on sépare de l'acide citrique du jus de fermentation.

9. Procédé selon la revendication 8, caractérisé en ce que l'on incorpore du méthanol au mélange de substrats à traiter en une quantité représentant au plus 4 % en poids desdits substrats.

10. Installation pour la fermentation en milieu solide d'un mélange de substrats glucidiques solides d'origine agricole, de substrats liquides d'origine agricole et

0140723

d'additifs, caractérisée en ce qu'elle comprend: une enceinte de fermentation cylindrique (4) entraînée en rotation autour de son axe et équipée intérieurement d'une vis sans fin périmétrale, de préférence à spires inclinées; des moyens pour introduire ledit mélange à l'extrémité inférieure de ladite enceinte; des moyens (3) pour évacuer le mélange fermenté à l'extrémité supérieure de ladite enceinte, des moyens (12) pour pulvériser de l'eau à l'intérieur de ladite enceinte et des moyens (11') pour y maintenir la température à un niveau prédéterminé.

11. Installation selon la revendication 10, caractérisée en ce que ledits moyens pour maintenir la température de ladite enceinte (4) à un niveau prédéterminé comprennent un système d'arrosage (11') de la partie extérieure de ladite enceinte.

0140723

Office européen
des brevets

**RAPPORT DE RECHERCHE EUROPEENNE**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | FR-A- 675 237 (F.J. CAHN)<br><br>* Page 1, ligne 27 - page 2, ligne 62; page 2, ligne 98 - page 3, ligne 12 *<br><br>--- | 1,4,8,<br>10 | C 12 M 1/10<br>C 12 M 1/16<br>C 12 P 7/48<br>C 12 N 1/16 //<br>(C 12 P 7/48<br>C 12 R 1:685)<br>(C 12 P 7/48<br>C 12 R 1:73 ) |
| Y | US-A-1 809 797 (F.J. CAHN)<br><br>* Page 2, lignes 55-87 *<br><br>--- | 1,4,8,<br>10 | (C 12 P 7/48<br>C 12 R 1:645) |
| A | FR-A-2 394 606 (INRA et al.)<br>* Revendication 1; figures; page 1, lignes 1-7; page 2, lignes 16-39 *<br><br>--- | 5 | |
| Y | US-A-4 223 094 (V.A. VASEEN)<br>* Figures; colonne 3, lignes 15-35 *<br><br>--- | 1 | |
| A | FR-A-2 502 639 (EGRETIER & CIE SA)<br>* Figures; revendications; page 1, lignes 1-7; page 2, lignes 16-39 *<br><br>--- | 1 | |
| A | DE-C- 292 586 (J. TAKAMINE)<br>* Figures; revendications *<br><br>----- | 1 | |

**DOMAINES TECHNIQUES RECHERCHES (Int. Cl.4)**

C 12 M
C 12 P
C 12 N

Le présent rapport de recherche a été établi pour toutes les revendications

| Lieu de la recherche | Date d'achèvement de la recherche | Examinateur |
|---|---|---|
| LA HAYE | 03-01-1985 | COUCKE A.O.M. |